(19) Europäisches Patentamt
European Patent Office
Office européen des brevets

(11) EP 0 530 336 B1

(12) EUROPÄISCHE PATENTSCHRIFT

(45) Veröffentlichungstag und Bekanntmachung des Hinweises auf die Patenterteilung:
**06.03.1996 Patentblatt 1996/10**

(51) Int Cl.6: **C07F 9/655**, B01J 31/28, C07F 9/572, C07F 9/6553, C07F 15/00

(21) Anmeldenummer: **92905551.5**

(22) Anmeldetag: **11.03.1992**

(86) Internationale Anmeldenummer:
**PCT/CH92/00049**

(87) Internationale Veröffentlichungsnummer:
**WO 92/16536 (01.10.1992 Gazette 1992/25)**

# (54) CHIRALE PHOSPHINE

CHIRAL PHOSPHINES

PHOSPHINES CHIRALES

(84) Benannte Vertragsstaaten:
**AT BE CH DE DK FR GB IT LI NL SE**

(30) Priorität: **15.03.1991 CH 805/91**
**05.03.1992 CH 697/92**

(43) Veröffentlichungstag der Anmeldung:
**10.03.1993 Patentblatt 1993/10**

(73) Patentinhaber: **F. HOFFMANN-LA ROCHE & CO. Aktiengesellschaft**
**CH-4002 Basel (CH)**

(72) Erfinder:
- **BROGER, Emil, Albin**
**CH-4312 Magden (CH)**
- **FORICHER, Joseph**
**F-68200 Mulhouse (FR)**
- **HEISER, Bernd**
**D-7854 Inzlingen (DE)**
- **SCHMID, Rudolf**
**CH-4142 Münchenstein (CH)**

(74) Vertreter: **Cottong, Norbert A. et al**
**Grenzacherstrasse 124**
**Postfach 3255**
**CH-4002 Basel (CH)**

(56) Entgegenhaltungen:
**EP-A- 0 151 282** **EP-A- 0 398 132**

Bemerkungen:
Die Akte enthält technische Angaben, die nach dem Eingang der Anmeldung eingereicht wurden und die nicht in dieser Patentschrift enthalten sind.

EP 0 530 336 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft neue, racemische und optisch aktive Phosphorverbindungen der allgemeinen Formel

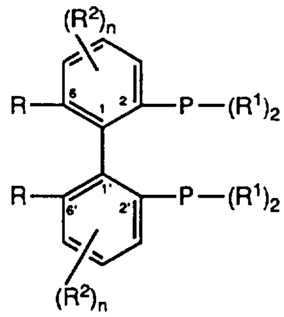

I

worin R niederes Alkyl, niederes Alkoxy oder eine geschützte Hydroxygruppe bedeutet, $R^1$ einen fünfgliedrigen Heteroaromaten bedeutet, $R^2$ für niederes Alkyl oder niederes Alkoxy steht und n die Zahl 0,1 oder 2 darstellt.

Die Erfindung betrifft ferner die Herstellung der Phosphorverbindungen der Formel I, sowie deren Verwendung für enantioselektive Reaktionen, wie z.B. asymmetrische Hydrierungen oder auch enantioselektive Wasserstoffverschiebungen in prochiralen, allylischen Systemen.

**Optisch aktive Phosphorverbindungen welche für obige Zwecke eingesetzt werden können, sind dem Fachmann bereits bekannt, so sind beispielsweise in EP-A 0 398132 (Alkoxybiphenyl-2,2'-diyl)bis ( diphenylphosphin) -Derivate beschrieben.**

Der Ausdruck "niederes Alkyl" bedeutet, im Rahmen der vorliegenden Erfindung, geradkettige oder verzweigte Alkylgruppen mit 1 bis 4 Kohlenstoffatomen wie Methyl, Aethyl, Propyl, Isopropyl, n-Butyl, Isobutyl und tert. Butyl. Der Ausdruck "niederes Alkoxy" bedeutet Gruppen, in denen der Alkylrest die vorhergehende Bedeutung hat. Als Schutzgruppen für die Hydroxy gruppe kommen, im Rahmen der vorliegenden Erfindung, insbesondere in Frage die üblichen Aether-bildenden Gruppen, wie z.B. Benzyl, Allyl, Benzyloxymethyl, niederes Alkoxymethyl oder auch 2-Methoxyäthoxymethyl und dergleichen. Der Ausdruck "fünfgliedriger Heteroaromat" steht, im Rahmen der vorliegenden Erfindung, für einen Substituenten der Formel

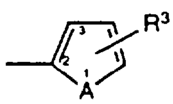 (a) 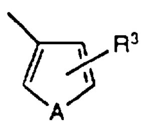 (b) 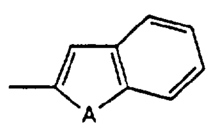 (c) 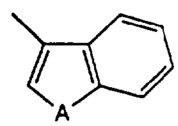 (d)

In den Substituenten der Formeln (a) bis (d) wiederum bedeutet A Sauerstoff, Schwefel oder -$NR^4$. Der Substituent $R^3$ bedeutet hier Wasserstoff, niederes Alkyl, insbesondere Methyl oder niederes Alkoxy, insbesondere Methoxy, und $R^4$ steht für niederes Alkyl, vorzugsweise Methyl.

Die Phosphorverbindungen der Formel I können sowohl in racemischer als auch in optisch aktiver Form vorliegen. Bevorzugte Verbindungen der Formel I sind solche, worin n für die Zahl 0 steht und R Methoxy, Methoxymethoxy oder Methyl bedeutet. Ferner sind auch noch diejenigen bevorzugt, worin $R^1$ 2- oder 3-Furyl oder 2-Thienyl darstellt. Besonders bevorzugte Verbindungen der Formel I sind:

(RS)-(6,6'-Dimethoxybiphenyl-2,2'-diyl)bis(di-2-furylphosphin),
(R)- oder (S)-(6,6'-Dimethoxybiphenyl-2,2'-diyl)bis(di-2-furylphosphin),
(RS)-(6,6'-Dimethoxybiphenyl-2,2'-diyl)bis(di-2-thienylphosphin),
(R)- oder (S)-(6,6'-Dimethoxybiphenyl-2,2'-diyl)bis(di-2-thienylphosphin),
(RS)-(6,6'-Dimethoxybiphenyl-2,2'-diyl)bis(di-3-furylphosphin),
(R)- oder (S)-(6,6'-Dimethoxybiphenyl-2,2'-diyl)bis(di-3-furylphosphin),
(RS)-(6,6'-Dimethylbiphenyl-2,2'-diyl)bis(di-2-furylphosphin),
(R)- oder (S)-(6,6'-Dimethylbiphenyl-2,2'-diyl)bis(di-2-furylphosphin),
(RS)-(6,6'-Dimethylbiphenyl-2,2'-diyl)bis(di-2-thienylphosphin),
(R)- oder (S)-(6,6'-Dimethylbiphenyl-2,2'-diyl)bis(di-2-thienylphosphin),
(RS)-[6,6'-Bis(methoxymethoxy)biphenyl-2,2'-diyl]bis(di-2-furylphosphin),
(R)- oder (S)-[6,6'-Bis(methoxymethoxy)biphenyl-2,2'-diyl]bis(di-2-furylphosphin),
(RS)-[6,6'-Bis(methoxymethoxylbiphenyl-2,2'-diyl]bis(di-2-thienylphosphin),
(R)- oder (S)-[6,6'-Bis(methoxyrmethoxy,)biphenyl-2,2'-diyl]bis(di-2-thienylphosphin)

(RS)-(6,6'-Dimethoxybiphenyl-2,2-diyl)bis[bis(5-methylfuran-2-yl)-phosphin,
(R) oder (S)-(6,6'-Dimethoxybiphenyl-2,2'-diyl)bis[bis(5-methylfuran-2-yl)-phosphin,
(RS)-(6,6'-Dimethoxybiphenyl-2,2'-diyl)bis[di-(2-benzo[b]furanyl)-phosphin],
(R)- oder (S)-(6,6'-Dimethoxybiphenyl-2,2'-diyl)bis[di-(2-benzo[b]furanyl)-phosphin],
(RS)-(6,6'-Dimethoxybiphenyl-2,2'-diyl)bis(dibenzo[b]thiophen-2-yl)-phosphin,
(R)- oder (S)-(6,6'-Dimethoxybiphenyl-2,2'-diyl)bis(dibenzo[b]thiophen-2-yl)phosphin,
(RS)-(6,6'-Dimethoxybiphenyl-2,2'-diyl)bis[bis(1-methylpyrrol-2-yl)-phosphin,
(R)- oder (S)-(6,6'-Dimethoxybiphenyl-2,2'-diyl)bis[bis(1-methylpyrrol-2-yl)phosphin.

Die erfindungsgemässen Verbindungen der Formel I können z.B. dadurch hergestellt werden, dass man eine racemische oder optisch aktive Verbindung der allgemeinen Formel

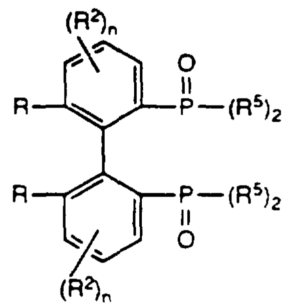

II

worin R, $R^2$ und n die obige Bedeutung haben und $R^5$ niederes
Alkoxy, Phenoxy, Benzyloxy, Chlor oder Brom bedeutet, mit einer Verbindung der Formel

$$R^1MgX \text{ oder } R^1Li$$

worin $R^1$ die obige Bedeutung hat und X Chlor, Brom oder Jod darstellt,

zu einer Verbindung der Formel

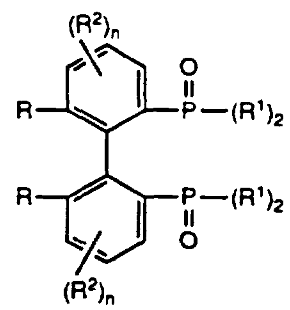

III

worin R, $R^1$, $R^2$ und n die obige Bedeutung haben,

umsetzt, und diese, gegebenenfalls nach Spaltung mittels O,O'-Dibenzoylweinsäure oder O,O'-Di-p-Toluylweblsäure in die (R)- und (S)-Form, zu einer Verbindung der Formel I reduziert.

Die Umsetzung einer Verbindung der Formel II mit $R^1MgX$ oder $R^1Li$ kann in an sich bekannter Weise erfolgen. Vorzugsweise erfolgt dies z.B. unter den üblichen Bedingungen einer Grignard-Reaktion.

Die Reduktion einer racemischen oder in (R)- oder (S)-Form vorliegenden Verbindung der Formel III kann in an sich bekannter Weise durchgeführt werden. Dies kann beispielsweise mit Silanen, wie z.B. Trichlorsilan, in einem aromatischen Kohlenwasserstoff, wie etwa in siedendem Xylol, oder auch in Acetonitril usw., zweckmässig in Gegenwart einer Hilfsbase, wie etwa Triäthylamin, oder vorzugsweise Tributylamin, erfolgen. Gewünschtenfalls kann diese Reduktion auch in einem Autoklaven unter Druck durchgeführt werden.

Die als Ausgangsmaterial verwendeten Verbindungen der Formel II können beispielsweise dadurch hergestellt werden, dass man eine Verbindung der Formel

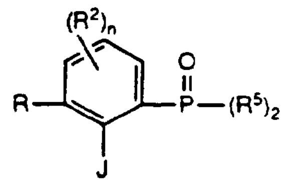

IV

worin R, $R^2$ und n die obige Bedeutung haben und $R^5$ ebenfalls obige Bedeutung hat, mit Ausnahme von Chlor oder Brom, einer Ullmann-Kopplung unterwirft, und dass man, gewünschtenfalls, eine so erhaltene, in der (RS)-Form vorliegende Verbindung der Formel

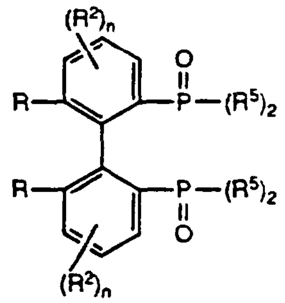

II

worin R, $R^2$, $R^5$ und n die Bedeutung aus Formel IV haben,

mittels O,O'-Dibenzoylweinsäure oder O,O'-Di-p-Toluylweinsäure in die (R)-und (S)-Form auftrennt, und dass man, gewünschtenfalls, in einer racemischen oder optisch aktiven Verbindung der Formel II, worin $R^5$ niederes Alkoxy darstellt, dieses durch Chlor oder Brom ersetzt.

Die Ueberführung einer Verbindung der Formel IV in eine, in (RS)-Form vorliegende Verbindung der Formel II erfolgt mittels einer Ullmann-Kopplung. Es handelt sich hierbei um eine an sich bekannte Reaktion, welche unter den hierfür üblichen Bedingungen durchgeführt werden kann. So kann diese Reaktion beispielsweise durch Erhitzen einer Verbindung der Formel IV in einem inerten organischen Lösungsmittel, wie z.B. N,N-Dimethylformamid, mit z.B. mit Jod aktiviertem Kupferpulver auf eine Temperatur von etwa 110°C bis etwa 200°C, durchgeführt werden. Gegebenenfalls kann die Reaktion auch ohne Lösungsmittel, d.h. in der Schmelze durchgeführt werden.

Die hier wiederum als Ausgangsmaterial verwendeten Verbindungen der allgemeinen Formel IV können, sofern R verschieden ist von niederem Alkyl, beispielsweise dadurch hergestellt werden, dass man eine Verbindung der allgemeinen Formel

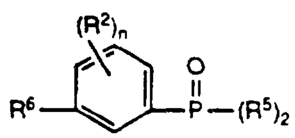

V

worin $R^2$, $R^5$ und n die Bedeutung von Formel IV haben, und $R^6$ niederes Alkoxy oder geschütztes Hydroxy bedeutet,

einer ortho-Lithiierungs-/Jodierungs-Reaktion unterwirft.

Die ortho-Lithiierung einer Verbindung der Formel V kann in an sich bekannter Weise erfolgen. Beispielsweise kann diese Reaktion durch Umsetzung einer Verbindung der Formel V mit einem Lithiumamid, z.B. Lithiumdiisopropylamid oder Lithium-2,2,6,6-tetramethylpiperidid in Tetrahydrofuran bei einer Temperatur unterhalb 0°C, vorzugsweise bei etwa -50° bis etwa -78°C, erfolgen. Die anschliessende Jodierung kann zweckmässig mit molekularem Jod, mit JCl oder JBr, ebenfalls in Tetrahydrofuran und ebenfalls bei einer Temperatur unterhalb -50°C, erfolgen.

Diejenigen Ausgangsmaterialien der Formel IV, worin R niederes Alkyl bedeutet, können beispielsweise ausgehend von einer Verbindung der Formel

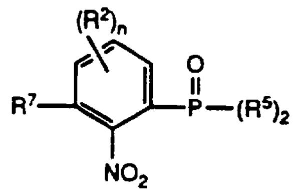

VI

worin $R^2$, $R^5$ und n die Bedeutung von Formel IV haben, und $R^7$ niederes Alkyl darstellt,

hergestellt werden.

Dies erfolgt zweckmässig durch allgemein bekannte Reduktion der Nitrogruppe zur Aminogruppe, z.B. mittels Wasserstoff in Gegenwart eines Katalysators wie etwa Pd/C, und anschliessende Diazotierung/Jodierung in an sich bekannter Weise.

Die Verbindungen der Formeln V und VI sind bekannte Verbindungen oder Analoge bekannter Verbindungen, welche leicht in an sich bekannter Weise hergestellt werden können Verbindungen V z.B. gemäss [J.J. Monagle et al., J. Org. Chem. 32, 2477 (1967)]. Verbindungen VI z.B. gemäss K.S. Petrakis et al., J. Am. Chem. Soc. 1987, 189, 2831.

Alternativ zum im vorhergehend Beschriebenen, können diejenigen Verbindungen der Formel II, worin R niederes Alkyl darstellt und $R^5$ von Chlor oder Brom verschieden ist, auch ausgehend von Verbindungen der Formel

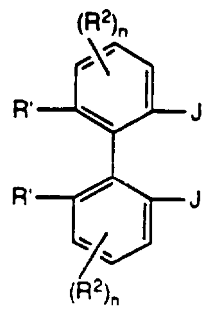

VII

worin $R^2$ und n obige Bedeutung haben und R' niederes Alkyl darstellt,

erhalten werden.

Dies kann in einfacher und an sich bekannter Weise erfolgen, z.B. durch Umsetzung einer Verbindung der Formel VII mit einer Verbindung der Formel

$$H\text{-}P\text{-}(R^5)_2$$
II
O

bzw. $P(OR^5)_2$

worin $R^5$ die obige Bedeutung hat mit Ausnahme von Chlor oder Brom,

in Gegenwart eines tert. Amins wie etwa Triäthylamin und eines Katalysators wie z.B. $Pd(P(Phenyl)_3)_4$, bzw. in Gegenwart eines Katalysators wie z.B. $PdCl_2$ oder auch $NiCl_2$..

Die Racematspaltung einer in der (RS)-Form vorliegenden Verbindung der Formel II L (worin $R^5$ von Chlor oder Brom verschieden ist) oder III mittels (-)- bzw. (+)-O,O'-Dibenzoylweinsäure (DBW) oder (-)- bzw. (+)-O,O'-Di-p-Toluylweinsäure (DTW) kann in für Racematspaltungen von Phosphinoxiden analoger Weise durchgeführt werden, und zwar obwohl dies gemäss Stand der Technik für die Verbindungen der Formel II eigentlich nicht zu erwarten war. Dies erfolgt zweckmässig in einem inerten organischen Lösungsmittel und bei einer Temperatur von etwa 0°C bis etwa 60°C. Als Lösungsmittel können hier insbesondere genannt werden Chloroform, Methylenchlorid, Essigsäureäthylester, Essigsäure-isopropylester, Aceton, Alkohole wie Methanol oder Aethanol und dergleichen, sowie auch Gemische davon.

Die so erhaltenen Addukte der Verbindungen der Formel II oder III mit (-)- bzw. (+)-DBW bzw. DTW können anschliessend, in zu Phosphinoxid-Addukten analoger Weise, mit einer anorganischen Base behandelt werden, wobei die jeweilige (R)- bzw- (S)-Form der Verbindungen der Formel II oder III freigesetzt werden.

Sofern in einer racemischen oder optisch aktiven Verbindung der Formel II $R^5$ niederes Alkoxy bedeutet, kann

dieses durch Chlor oder Brom ersetzt werden. Diese Substitution kann in an sich bekannter Weise erfolgen, beispielsweise durch Umsetzung mit Thionylchlorid, Tionylbromid oder Phosphorpentachlorid in einem inerten organischen Lösungsmittel.

Sämtliche vorhergehend erwähnten Reaktionen - mit Ausnahme der Racematspaltung - werden zweckmässig unter Inertgas, wie z.B. Argon oder Stickstoff, durchgeführt.

Die erfindungsgemässen Phosphorverbindungen der Formel I bilden Komplexe mit Uebergangsmetallen wie etwa Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium- und Rhodium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel I sind neu und ebenfalls Gegenstand der vorliegenden Erfindung.

Die in Frage stehenden Komplexe können in an sich bekannter Weise hergestellt werden, z.B. indem man eine Verbindung der Formel I mit einer Verbindung, welche ein Metall der Gruppe VIII abgeben kann, in einem geeigneten, inerten organischen oder wässrigen Lösungsmittel umsetzt. Als geeignete, z.B. Rhodium abgebende Verbindungen können beispielsweise genannt werden, organische Rhodium-Komplexe mit Aethylen, Propylen und dergleichen, sowie mit bis-Olefinen, z.B. (Z,Z)-1,5-Cyclooctadien, 1,5-Hexadien, Bicydo[2.2.1]hepta-2,5-dien oder mit weiteren Dienen, welche mit Rhodium leicht lösliche Komplexe bilden. Bevorzugte, Rhodium abgebende Verbindungen sind z.B. Di-μ-chioro-bis[$\eta^4$-(Z,Z)-1,5-cydooctadien]dirhodium(I), Di-μ-chloro-bis[$\eta^4$-norbomadien]dirhodium(I), Bis[η4-(Z,Z)-1,5-cydooctadien]-rhodium-tetrafluorborat oder Bis[$\eta^4$-(Z,Z)-cyclooctadien]rhodium-perchlorat. Als Iridium abgebende Verbindung kann beispielsweise Di-μ-chloro-bis[$\eta^4$-(Z,Z)-1,5-cyclooctadien]diiridum(I) genannt werden.

Die in Frage stehenden Ruthenium-Komplexe können z.B. durch folgende Formel dargestellt werden

$$Ru(Z)_2L \qquad VIII$$

worin Z Halogen oder die Gruppe A-COO, A niederes Alkyl, Aryl, halogeniertes niederes Alkyl oder halogeniertes Aryl und L einen chiralen Diphosphinliganden der Formel I darstellen.

Diese Komplexe können im Prinzip in an sich bekannter Weise hergestellt werden. Zweckmässig und bevorzugt werden Ruthenium-Komplexe beispielsweise dadurch hergestellt, dass man einen Komplex der Formel

$$[Ru(Z^1)_2L^1_m]_p \cdot (H_2O)_q \qquad IX$$

worin $Z^1$ Halogen oder eine Gruppe $A^1$-COO, $A^1$ niederes Alkyl oder halogeniertes niederes Alkyl, $L^1$ einen neutralen Liganden, m die Zahl 1, 2 oder 3, p die Zahl 1 oder 2 und q die Zahl 0 oder 1 darstellen,

mit einem chiralen Diphosphinliganden der Formel I umsetzt, oder, dass man einen Ruthenium-Komplex der Formel

$$Ru(CF_3COO)_2L \qquad X$$

worin L einen chiralen Diphosphinliganden der Formel I darstellt, mit einem das Anion Z abgebenden Salz, worin Z obige Bedeutung hat, umsetzt.

Der Ausdruck "neutraler Ligand" bedeutet im Rahmen der vorliegenden Erfindung, einen leicht austauschbaren Liganden wie etwa ein Diolefin, z.B. Norbornadien, (Z,Z)-1,5-Cyclooctadien usw., oder auch ein Nitril wie Acetonitril, Benzonitril und dergleichen. Falls m die Zahl 2 oder 3 darstellt, können die Liganden gleich oder auch verschieden sein.

Die Rutheniumkomplexe der Formel IX sind bekannte Substanzen oder Analoge bekannter Substanzen, welche leicht in zur Herstellung der bekannten analoger Weise erhalten werden können, beispielsweise gemäss Albers, M.O. et al., J. Organomet. Chem. 272, C62-C66 (1984).

Die Umsetzung eines Rutheniumkomplexes der Formel IX mit einem chiralen Diphosphinliganden der Formel I kann in an sich bekannter Weise durchgeführt werden. Diese Reaktion kann zweckmässig in einem inerten organischen Lösungsmittel erfolgen. Als Beispiele derartiger Lösungsmittel können genannt werden, z.B. Aether wie Tetrahydrofuran oder Dioxan, Ketone wie etwa Aceton, niedere Alkohole wie etwa Methanol, Aethanol usw., halogenierte Kohlenwasserstoffe wie Methylenchlorid, Chloroform und dergleichen, oder auch Gemische derartiger Lösungsmittel. Die Reaktion kann zudem bei einer Temperatur zwischen etwa 0°C und etwa 100°C, und vorzugsweise zwischen etwa 15°C und etwa 60°C erfolgen, jedoch unter striktem Ausschluss von Sauerstoff.

Die Umsetzung eines Rutheniumkomplexes der Formel X (erhältlich aus einem Komplex der Formel IX) mit einem

das Anion Z enthaltenden Salz kann in an sich bekannter Weise erfolgen. Der Ausdruck "ein das Anion Z abgebendes Salz" bedeutet im Rahmen der vorliegenden Erfindung beispielsweise Ammoniumsalze, Alkalimetallsalze oder andere geeignete Metallsalze. Um die Löslichkeit derartiger Salze zu verbessern, können in gewissen Fällen auch Kronenäther oder dergleichen zugesetzt werden.

Wie bereits eingangs erwähnt, sind die erfindungsgemässen Phosphorverbindungen, in Form von Komplexen mit Metallen der Gruppe VIII, und insbesondere Ruthenium, u.a. verwendbar für asymmetrische Hydrierungen. Als besonders geeignete Substrate können in diesem Zusammenhang insbesondere genannt werden, Allylalkohole wie z.B. Geraniol, 6,7-Dihydrogeraniol, 6,7-Dihydrofarnesol, 6,7,10,11-Tetrahydrofarnesol und dergleichen, sowie auch β-Ketoester wie z.B. Acetessigsäuremethyl- oder äthylester usw.

Bei der Durchführung derartiger Hydrierungen, können diese Komplexe zuerst hergestellt und dann zu einer Lösung der zu hydrierenden Substanz gegeben werden. Alternativ können sie jedoch auch in situ hergestellt werden, z.B. auch in Gegenwart einer zu hydrierenden Substanz.

Die asymmetrische Hydrierung kann in einem geeigneten, unter den Reaktionsbedingungen inerten organischen Lösungsmittel erfolgen. Als derartige Lösungsmittel können insbesondere genannt werden, niedere Alkohole wie z.B. Methanol oder Aethanol, oder Gemische derartiger Alkohole mit halogenierten Kohlenwasserstoffen wie Methylenchlorid, Chloroform und dergleichen, oder mit cydischen Aethern wie Tetrahydrofuran oder Dioxan, und dergleichen.

Das Verhältnis von Ruthenium zu Ligand L liegt zweckmässig zwischen etwa 0,5 und etwa 2 Mol, vorzugsweise bei etwa 1 Mol Ruthenium pro Mol Ligand. Das Verhältnis von Ruthenium, in den Komplexen der Formel VIII, zu den zu hydrierenden Substanzen liegt zweckmässig zwischen etwa 0,0005 und eta 1 Mol %, vorzugsweise zwischen etwa 0,002 und etwa 0,1 Mol %.

Die asymmetrische Hydrierung mit den Komplexen der Formel VIII erfolgt zweckmässig bei einer Temperatur von etwa 0°C bis etwa 100°C, in Abhängigkeit des verwendeten Substrates. Diese Hydrierung erfolgt zweckmässig auch unter Druck, vorzugsweise bei einem Druck von etwa 5 bis etwa 200 bar, besonders bevorzugt von etwa 30 bis etwa 100 bar.

Weiterhin sind die erwähnten Katalysatoren, wie eingangs erwähnt, verwendbar für enantioselektive Wasserstoffverschiebungen in prochiralen allylischen Systemen. Besonders interessant sind sie z.B. im Zusammenhang mit der Herstellung von optisch aktiven Verbindungen der allgemeinen Formel

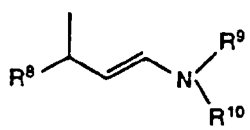

XI

worin $R^8$ geschütztes Hydroxymethyl oder einen Rest der Formel

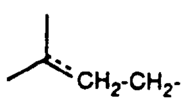

oder

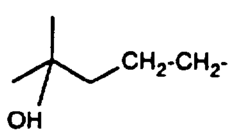

darstellt, worin die gestrichelte Linie eine zusätzliche Bindung darstellen kann, und $R^9$ und $R^{10}$ niederes Alkyl (1-7 C-Atome) bedeuten,

ausgehend von Verbindungen der allgemeinen Formel

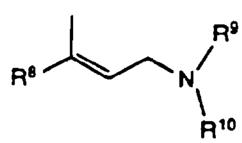

XII

worin $R^8$, $R^9$ und $R^{10}$ die obige Bedeutung haben.

Die Verbindungen XI, bzw. die daraus durch Hydrolyse erhaltenen Aldehyde, sowie die von letzteren abgeleiteten Säuren und Alkohole sind z.B. von Interesse als Zwischenprodukte bei der Synthese der Seitenketten der Vitamine E und $K_1$.

Zur Durchführung der erwähnten Wasserstoffverschiebungen, können die Phosphorverbindungen der Formel I als solche, in einer Lösung einer zu behandelnden Verbindung, mit einer z.B. Rhodium oder Iridum abgebenden Verbindung in Kontakt gebracht werden. Andererseits können die Phosphorverbindungen der Formel I zunächst in einem geeigneten Lösungsmittel mit einer z.B. Rhodium oder Iridum abgebenden Verbindung zu dem entsprechenden Katalysator-Komplex umgesetzt werden, und dieser dann zu einer Lösung einer zu behandelnden Verbindung gegeben werden, wobei die letztere Methode bevorzugt ist.

Sowohl die Umsetzung der Phosphorverbindungen der Formel I mit einer z.B. Rhodium oder Iridum abgebenden Verbindung, wie auch die erwähnten Wasserstoffverschiebungen können in geeigneten, unter den Reaktionsbedingungen inerten organischen Lösungsmitteln durchgeführt werden. Als solche können insbesondere genannt werden, niedere Alkanole wie z.B. Methanol oder Aethanol, aromatische Kohlenwasserstoffe wie Benzol oder Toluol, cyclische Aether wie Tetrahydrofuran oder Dioxan, Ester wie z.B. Essigester, oder auch Gemische hiervon, und dergleichen. Weiterhin können die Komplexbildungen auch noch in wässrigem Medium oder in Dichlormethan durchgeführt werden.

Das Verhältnis zwischen z.B. Rhodium oder Iridum und den Liganden der Formel I liegt zweckmässig zwischen etwa 0,05 und etwa 5 Mol, vorzugsweise zwischen etwa 0,5 und etwa 2 Mol Metall pro Mol Ligand der Formel I.

Die Menge an Metall, in den Komplexen mit den Liganden der Formel I, bezogen auf die zu behandelnden Verbindungen, liegt zweckmässig zwischen etwa 0,005 und etwa 0,5 Mol %, vorzugsweise zwischen etwa 0,01 und etwa 0,2 Mol %.

Die erwähnten Wasserstoffverschiebungen unter Verwendung von Metall-Komplexen mit den Liganden der Formel I können zweckmässig in einem inerten, organischen Lösungsmittel und bei einer Temperatur von etwa Raumtemperatur bis etwa 130°C durchgeführt werden. Diese Reaktion erfolgt vorzugsweise bei erhöhter Temperatur, d.h. je nach verwendetem Lösungsmittel entweder bei Rückflusstemperatur des Reaktionsgemisches oder in einem geschlossenen Gefäss unter Druck.

In Analogie zur Herstellung und Verwendung der Verbindungen der Formel I, können ebenfalls auch Verbindungen des Binaphthyl-Typus der folgenden Formel

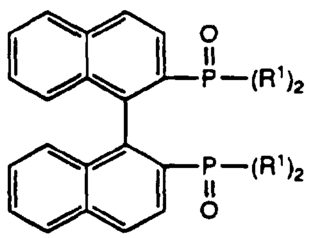

XIII

worin $R^1$ obige Bedeutung hat,

hergestellt und verwendet werden. Die Binaphthylringe können hierbei in üblicher Weise substituiert sein.

Die folgenden Beispiele dienen zur fllustrierung der Erfindung und stellen in keiner Weise irgendeine Beschränkung dar. In diesen Beispielen haben die gewählten Abkürzungen folgende Bedeutung:

DC: Dünnschichtchromatographie
DBW: O,O'-Dibenzoylweinsäure
THF: Tetrahydrofuran
RT: Raumtemperatur

Alle Temperaturen sind in °Celsius angegeben.

Biespiel 1

a) Zu einer aus 1,5 ml 2-Jodfuran (67% rein) und 0,30 g (12,3 mMol) Magnesiumspänen in 10 ml Tetrahydrofuran hergestellten Grignardlösung wurde bei Raumtemperatur innerhalb von 15 Minuten eine Lösung von 0,30 g (0,442 mMol) (R)-(6,6'-Dimethoxybiphenyl-2,2'-diyl)bis(phosphonsäurediphenylester) in 10 ml Tetrahydrofuran getropft. Nach beendeter Zugabe wurde 1 Stunde auf 40° erhitzt. Zur Aufarbeitung wurde mit 50 ml ges. $NH_4Cl$-Lösung und 50 ml Essigsäureäthylester versetzt, die Phasen getrennt, und die organische Phase wurde mit ges. NaCl-Lösung gewaschen, über $MgSO_4$ getrocknet, filtriert und eingeengt. Der Rückstand wurde in der minimalen Menge $CH_2Cl_2$ gelöst und die Lösung auf eine Säule von 50 g Kieselgel aufgetragen. Elution mit Essigsäureäthylester und dann mit Tetrahydrofuran lieferte 0,20 g eines Feststoffes, welcher aus 10 ml tert. Butylmethyläther umkristallisiert wurde. Man erhielt 0,16 g (R)-(6,6'-Dimethoxybiphenyl-2,2'-diyl)bis(di-2-furylphosphinoxid) als gelbliche Kristalle, Smp. 272° (Thermoanalyse); $[\alpha]_D{}^{20} = +89{,}6$ (c = 1,0, $CHCl_3$).

b) Ein 0,5 1 Vierhalssulfierkolben, versehen mit Kühler, Thermometer, Tropftrichter und mechanischem Rührer, wurde unter Argon mit 2,90 g (5,0 mMol) (R)-(6,6'-Dimethoxybiphenyl-2,2'-diyl)bis(di-2-furylphosphinoxid), 10 ml (41,9 mMol) Tributylamin, 60 ml Xylol-Isomerengemisch und 4,0 ml (5,37 g, 39,6 mMol) Trichlorsilan beschickt. Die

müchig-weisse Mischung wurde 4 Stunden unter Rückfluss gekocht, wobei eine beinahe durchscheinende Lösung entstand. Nach Abkühlung wurden unter gutem Rühren 100 ml deoxygenierte 30% Natronlauge so zugetropft, dass die Innentemperatur 70° nicht überstieg, und die Mischung wurde noch 1 Stunde bei 70° gerührt. Nach Zugabe von $H_20$ und $CH_2Cl_2$ wurden die Phasen getrennt, und die organische Phase wurde mit 2 x 50 ml 30% Natronlauge, $H_2O$, ges. $NH_4Cl$-und ges. NaCl-Lösung gewaschen, über $MgSO_4$ getrocknet, filtriert und eingedampft. Das erhaltene weisse Pulver (4,40 g) wurde in $CH_2Cl_2$ gelöst, die Lösung mit Aethanol versetzt, und das $CH_2Cl_2$ wurde am Rotationsverdampfer abgedampft. Der ausgefallene Festkörper wurde abfiltriert, mit Aethanol und Pentan gewaschen und am Hochvakuum (~ 10 Pa) während 1 Stunde bei 100° getrocknet. Man erhielt 2,50 g (R)-(6,6'-Dimethoxybiphenyl-2,2'-diyl)bis(di-2-furylphosphin) als gelbliche Kristalle; Smp. 176°; $[\alpha]_D,^{20}$ = +6,9 (c = 1, $CHCl_3$).

In zum Vorhergehenden analoger Weise wurden auch folgende Verbindungen hergestellt:

(S)-(6,6'-Dimethoxybiphenyl-2,2'-diyl)bis(di-2-furliphosphinoxid). Smp. 270° (Thermoanalyse). $[\alpha]_D,^{20}$ = -87,9 (c = 1, $CHCl_3$).
(S)-(6,6'-Dimethoxybiphenyl-2,2'-diyl)bis(di-2-furylphosphin). Smp. 175° (Thermoanalyse). $[\alpha]_D,^{20}$ = -7,6 (c = 1, $CHCl_3$).

Der als Ausgangsmaterial verwendete (R)-(6,6'-Dimethoxybiphenyl-2,2'-diyl)bis(phosphonsäure-diphenylester) bzw. die entsprechende (S)-Verbindung wurden wie folgt hergestellt:

a) In einem 1 l Vierhalssulfierkolben, versehen mit Kühler, Thermometer, Rührer und Aufsatz für Inertgasbehandlung, wurden unter Argon 76,5 g (0,122 Mol) (2-Jod-3-methoxyphenyl)phosphonsäure-diphenylester (75% rein) und 25,0 g (0,393 Mol) aktiviertes Kupferpulver vorgelegt und 200 ml N,N-Dimethylformamid zufliessen gelassen. Die dunkelbraune Suspension wurde 1 Stunde auf 140° erhitzt (Oelbadtemperatur), wonach gemäss DC-Analyse vollständiger Umsatz eingetreten war. Das abgekühlte Reaktionsgut wurde mit etwas Methylenchlorid in einen Rundkolben transferiert und am Rotationsverdampfer bei 70° zur Trockene eingedampft. Der Rückstand wurde mit 200 ml Methylenchlorid versetzt, die Mischung gut durchgerührt und filtriert, und der Filterrückstand mit 100 ml Methylenchlorid gewaschen. Das Filtrat wurde dreimal mit 100 ml ges. $NH_4Cl$-Lösung gewaschen, wobei bei der ersten Waschoperation von wenig gebildetem Festkörper filtriert wurde, und anschliessend über $MgSO_4$ getrocknet, filtriert und eingeengt. Nach Trocknung am Hochvakuum (- 10 Pa) während 2 Stunden bei 80° erhielt man 59,6 g rohen (RS)-(6,6'-Dimethoxybiphenyl-2,2'-diyl)bis(phosphonsäurediphenylester).

ba) In einem 1 l Rundkolben wurde eine Lösung von 59,6 g des rohen, gemäss a) erhaltenen Diphenylesters in 50 ml Dichlormethan vorgelegt und mit einer Lösung von 35,8 g (0,10 Mol) (-)-O,O'-Dibenzoyl-L-weinsäure in 100 ml Essigsäureäthylester versetzt. Die Lösung wurde dann am Rotationsverdampfer bei 600 mbar eingedampft, wobei das $CH_2Cl_2$ abdestillierte und ein weisser Festkörper ausfiel. Dieser wurde abgenutscht, gewaschen mit dreimal 20 ml Essigsäureäthylester und 20 ml Hexan und am Hochvakuum (~ 10 Pa) getrocknet. Man erhielt 21,8 g (R) -Diphenylester/(-)-DBW-Addukt als weisses Pulver. $[\alpha]_D,^{20}$ = -95,6 (c = 1 in Aethanol).

Die Mutterlaugen und Waschlösungen wurden zur Gewinnung des anderen Enantiomeren beiseite gestellt.

bb) Das gemäss ba) erhaltene Material wurde in einem 1 l Erlenmeyer mit Magnetrührer mit 100 ml Dichlormethan, 50 ml ges. $NaHCO_3$-Lösung und 50 ml entionisiertem Wasser verrührt, bis aller Festkörper in Lösung gegangen war (30 Minuten). Die Phasen wurden getrennt, und die organische Phase wurde zweimal mit 100 ml halbges. $NaHCO_3$-Lösung, 50 ml entionisiertem Wasser und 50 ml ges. NaCl-Lösung gewaschen, über $MgSO_4$ getrocknet, filtriert und eingedampft. Der ölige Rückstand wurde mit 20 ml tert.-Butylmethyläther versetzt, wobei Kristallisation eintrat. Nach Eindampfen und Trocknen am Hochvakuum (~ 10 Pa) während 1 Stunde bei 60° erhielt man 13,8 g (R)-(6,6'-Dimethoxybiphenyl-2,2'-diyl)bis(phosphonsäure-diphenylester) als weisse Kristalle. Smp. 125-125,5°; $[\alpha]_D,^{20}$ = -18,9 (c = 1 in $CHCl_3$).

ca) In einem 1 1 Rundkolben wurden die Mutterlaugen und Waschlösungen aus ba) eingedampft. Der Rückstand wurde in 100 ml Dichlormethan aufgenommen und die Lösung während 30 Minuten mit 50 ml ges. $NaHCO_3$-Lösung und 50 ml entionisiertem Wasser verrührt. Die Phasen wurden getrennt, und die organische Phase wurde mit 100 ml halbges. $NaHCO_3$-Lösung, 50 ml entionisiertem Wasser und 50 ml ges. NaCl-Lösung gewaschen, über $MgSO_4$ getrocknet, filtriert und eingeengt. Das erhaltene braune Oel wurde in 50 ml Dichlormethan aufgenommen, und die Lösung wurde mit einer Lösung von 18,0 g (0,050 Mol) (+)-O,O'-Dibenzoyl-D-weinsäure in 100 ml Essigsäureäthylester versetzt. Die Lösung wurde dann am Rotationsverdampfer bei 600 mbar aufkonzentriert, wobei das $CH_2Cl_2$ abdestillierte und ein weisser Festkörper ausfiel. Dieser wurde abgenutscht, dreimal mit 20 ml Essigsäureäthylester

und 20 ml Hexan gewaschen und am Hochvakuum (- 10 Pa) getrocknet. Man erhielt 22 g (S)-Diphenylester/(+) -DBW-Addukt als leicht gelbliches Pulver. $[\alpha]_D,^{20}$ = +96 (c = 1 in Aethanol).

cb) Das gemäss ca) erhaltene Material wurde wie in bb) beschrieben aufgearbeitet. Man erhielt 13,9 g (S)-(6,6'-Dimethoxybiphenyl-2,2'-diyl)bis(phosphonsäure-diphenylester) als weisse Kristalle. Smp. 124-125°; $[\alpha]_D,^{20}$ = +18,7 (c =1 in $CHCl_3$).

d) Der als Ausgangsmaterial verwendete (2-Jod-3-methoxyphenyl)-phosphonsäure-diphenylester wurde wie folgt hergestellt:

daa) In einem 0,5 1 Vierhalskolben, versehen mit Rührer, Kühler, Thermometer und Aufsatz für Inertgasbehandlung, wurde unter Argon eine Aufschlämmung von 13,0 g (0,535 Mol) Magnesiumspänen in 50 ml getrocknetem Tetrahydrofuran vorgelegt. Hierzu wurde eine Lösung von 93,5 g (0,50 Mol) 3-Bromanisol in 200 ml getrocknetem Tetrahydrofuran innerhalb von 90 Minuten so zugetropft, dass die Reaktionstemperatur 35° nicht überstieg. Nach erfolgter Zugabe wurde zur Vermeidung des Ausfallens des Grignardreagens mit zusätzlichen 150 ml getrocknetem Tetrahydrofuran verdünnt.

dab) In einem 1,5 1 Vierhalskolben, versehen mit Rührer, Thermometer, Aufsatz für Inertgasbehandlung und $CO_2$/Aceton-Kühlbad, wurden 259,8 g (0,967 Mol) Diphenyl-chlorphosphat und 200 ml getrocknetes Tetrahydrofuran vorgelegt, und die Lösung auf -78° gekühlt. Dazu wurde nun innerhalb 2 Stunden die Lösung des gemäss baa) bereiteten Grignardreagens so zugetropft, dass die Reaktionstemperatur -70° nicht überstieg. Nach beendeter Zugabe liess man über Nacht unter Rühren auf Raumtemperatur erwärmen. Die Reaktionsmischung, welche etwas feinen weissen Niederschlag enthielt, wurde in ein 10 l Rührgefäss auf ein Gemisch von 2l Eiswasser, 2l ges. $NaHCO_3$-Lösung und 1 l Diäthyläther gegossen. Nach kräftigem Durchrühren während 10 Minuten wurde die wässrige Phase abgetrennt und die organische Phase nacheinander mit 1l ges. $NaHCO_3$-Lösung, 200 ml 25% Ammoniak, 100 ml 25% Ammoniak und dreimal mit 500 ml ges. NaCl-Lösung gewaschen. Nach Trocknung über $MgSO_4$ wurde eingedampft, der Rückstand in 11 Diäthyläther aufgenommen, und die Lösung bei 0° über Nacht stehengelassen. Der dabei ausgeschiedene weisse Festkörper (12 g) wurde durch Filtration entfernt und verworfen. Das Filtrat wurde eingedampft, am Hochvakuum (~ 10 Pa) getrocknet, der erhaltene Rückstand (163 g gelbes Oel) in 300 ml Hexan/Toluol 1:1 aufgenommen und die Lösung über 500 g Kieselgel filtriert. Durch Elution mit zuerst 3l Hexan und 81 Hexan/Essigsäureäthylester 9:1 und danach mit 2 l Hexan/Essigsäureäthylester 4:1 und 2l Hexan/Essigsäureäthylester 7:3 erhielt man nach Trocknung im Hochvakuum (- 10 Pa) während 1 Stunde bei 40° 118 g (3-Methoxyphenyl)phosphonsäure-diphenylester als leicht gelbliches Oel.

db) In einem 1,5 l Vierhalskolben, versehen mit Rührer, Thermometer, Aufsatz für Inertgasbehandlung, Tropftrichter mit Druckausgleich und $CO_2$/Aceton-Kühlbad, wurden 300 ml getrocknetes Tetrahydrofuran vorgelegt. Hierzu wurden 70 ml (0,412 Mol) 2,2,6,6-Tetramethylpiperidin mittels einer Spritze zugefügt, und die Lösung wurde auf -78° abgekühlt. In den Tropftrichter wurden via eine Stahlkanüle 210 ml (0,336 Mol) 1,6N Butyllithium-Lösung in Hexan eingefüllt. Die Butyllithium-Lösung wurde innerhalb von ca. 10 Minuten in das Reaktionsgefäss eingetropft, wobei die Temperatur bis auf ca. -50° anstieg und ein weisser Niederschlag gebildet wurde. Das $CO_2$/Aceton-Kühlbad wurde durch ein Eis/Aethanol-Bad ersetzt, und die Reaktionsmischung wurde während 30 Minuten bei ca. -15° gerührt, dann wieder auf -78° gekühlt.

In einem separaten 1 1 Rundkolben wurden unter Argon 250 ml getrocknetes Tetrahydrofuran und 95,2 g (0,280 Mol) (3-Methoxyphenyl)phosphonsäure-diphenylester (Material aus dab) vorgelegt, und die Lösung wurde auf -78° abgekühlt. Diese Lösung wurde nun via eine Stahlkanüle innerhalb von ca. 10 Minuten in die obige Reaktionsmischung einfliessen gelassen, wobei die Temperatur bis auf ca. -68° anstieg und eine durchscheinende, caramelfarbene Lösung entstand. Diese wurde noch 30 Minuten bei -78° gerührt.

In einem separaten 250 ml Schlenkrohr wurde unter Argon eine Lösung von 71,06 g (0,280 Mol) Jod in 150 ml getrocknetem. Tetrahydrofuran hergestellt, und die Lösung wurde via Stahlkanüle in den Tropftrichter der Reaktionsapparatur transferiert. Die Reaktionsmischung wurde nun innerhalb 15 Minuten durch rasches Zutropfen der Jodlösung titriert, wobei die Reaktionstemperatur bis auf -65° anstieg. Nach Zutropfen von ca. 145 ml der ca. 170 ml Jodlösung, als eine rote Färbung der Reaktionsmischung bestehen blieb, wurde die Zugabe abgebrochen und man liess auf 0° erwärmen. Dann wurde die Reaktionsmischung mit 150 ml einer Lösung von 100 g Natriumthiosulfat-Pentahydrat in 200 ml entionisiertem Wasser versetzt, kräftig durchgerührt, und anschliessend mit 100 ml ges. $NaHCO_3$-Lösung versetzt. Das Zweiphasensystem wurde zur Entfernung von gebildetem Niederschlag filtriert und die Phasen getrennt. Die wässrige Phase wurde einmal mit 250 ml Essigsäureäthylester rückextrahiert, und die vereinigten organischen Phasen wurden mit 250 ml ges. NaCl-Lösung gewaschen, über $MgSO_4$ getrocknet und eingedampft. Der Rückstand wurde

nochmals in 500 ml Essigsäureäthylester aufgenommen, und die Lösung wurde dreimal mit 250 ml entionisiertem Wasser und mit 250 ml ges. NaCl-Lösung gewaschen, über $MgSO_4$ getrocknet, filtriert und eingeengt. Der Rückstand (118 g gelbes Oel) wurde in 170 ml Toluol aufgenommen und die Lösung mit 115 ml Hexan versetzt, wobei ein weisser Niederschlag ausfiel. Dieser wurde durch Filtration entfernt und das Filtrat wurde auf eine Säule von 450 g Kieselgel aufgetragen. Zunächst wurden mit 2l Hexan/Essigsäureäthylester 9:1 und 3l Hexan/Essigsäureäthylester 8:2 Nebenprodukte eluiert. Anschliessend wurden mit 2l Hexan/Essigsäureäthylester 7:3 die das Endprodukt enthaltenden Fraktionen eluiert. Nach Eindampfen und Trocknen am Hochvakuum (~ 10 Pa) während 1 Stunde bei 60° wurden 76,5 g eines orangen Oels erhalten. Dieses bestand gemäss $^1$H-NMR Analyse aus 75 Mol% (2-Jod-3-methoxyphenyl)phosphonsäure-diphenylester.

Beispiel 2

In zu Beispiel 1 analoger Weise wurden folgende Verbindungen hergestellt:

(R)-(6,6'-Dimethoxybiphenyl-2,2'-diyl)bis(di-2-thienylphosphinoxid). Smp. 337° (Thermoanalyse). $[\alpha]_D$,$^{20}$ = +141,3 (c = 1, $CHCl_3$).
(R)-(6,6'-Dimethoxybiphenyl-2,2'-diyl)bis(di-2-thienylphosphin). Smp. 230° (Thermoanalyse). $[\alpha]_D$,$^{20}$ = +147,5 (c = 1, $CHCl_3$).
(S)-(6,6'-Dimethoxybiphenyl-2,2'-diyl)bis(di-2-thienylphosphinoxid). Smp. 338° (Thermoanalyse) . $[\alpha]_D$,$^{20}$ = -140,7 (c = 1, $CHCl_3$).
(S)-(6,6'-Dimethoxybiphenyl-2,2'-diyl)bis(di-2-thienylphosphin). Smp. 228° (Thermoanalyse). $[\alpha]_D$,$^{20}$ = -158,5 (c = 1, $CHCl_3$).

Beispiel 3

In zu Beispiel 1 analoger Weise wurden folgende Verbindungen hergestellt:

(RS)-(6,6'-Dimethoxybiphenyl-2,2'-diyl)bis(di-3-furylphosphinoxid). Smp. 226-227°.
(RS)-(6,6'-Dimethoxybiphenyl-2,2'-diyl)bis(di-3-furylphosphin).
(R)-(6,6'-Dimethoxybiphenyl-2,2'-diyl)bis(di-3-furylphosphinoxid). Smp. 230-233°; $[\alpha]_D$,$^{20}$ = +90,3 (c = 1, $CHCl_3$).
(R)-(6,6'-Dimethoxybiphenyl-2,2'-diyl)bis(di-3-irylphosphin). Smp. 128-129°; $[\alpha]_D$,$^{20}$ = +41,3 (c = 1, $CHCl_3$).
(S)-(6,6'-Dimethoxybiphenyl-2,2'-diyl)bis(di-3-furylphosphinoxid). $[\alpha]_D$,$^{20}$ = -87 (c = 1, $CHCl_3$).
(S)-(6,6'-Dimethoxybiphenyl-2,2'-diyl)bis(di-3-furylphwsphin). Smp. 135°; $[\alpha]_D$,$^{20}$ = -41,3 (c = 1, $CHCl_3$).

Beispiel 4

In einem 1 l Sulfierkolben, versehen mit Kühler, Thermometer, Rührer und Aufsatz für Inertgasbehandelung, wurden unter Argon 0,6677 g (1,1 mMol) (RS)-(6,6'-Dimethylbiphenyl-2,2'-diyl)bis(di-2-thienylphosphinoxid), 1,49 g (11 mMol) $HSiCl_3$ und 0,445 g (4,4 mMol) Triäthylamin in 15 ml Xylol vorgelegt und 4 Stunden unter Rückfluss gerührt. Die erhaltene klare Lösung wurde vorsichtig mit 5 ml 30%iger NaOH-Lösung versetzt, wobei die Reaktionstemperatur auf ~50° anstieg. Die organische Phase wurde abgetrennt und nacheinander mit 5 ml 30%iger NaOH-Lösung und 10 ml $H_2O$ gewaschen und über $Na_2SO_4$ getrocknet. Nach Eindampfen wurde ein weisses Pulver erhalten, welches mit Pentan gewaschen und bei - 10 Pa getrocknet wurde. Man erhielt 0,44 g (RS)-(6,6'-Dimethylbiphenyl-2,2'-diyl)bis(di-2-thienylphosphin). Smp. 177-178°.

Das als Ausgangsmaterial verwendete (RS)-(6,6'-Dimethylbiphenyl-2,2'-diyl)bis(di-2-thienylphosphinoxid) wurde wie folgt hergestellt:

a) In einem Schlenkrohr wurden 20 g (46,07 mmol) (RS)-2,2'-Dijod-6,6'-dimethylbiphenyl, 23,72 g (101,3 mMol) Diphenylphosphit, 12 g (119 mMol) Triäthylamin und 2,62 g (2,26 mMol) $Pd(PPh_3)_4$ unter Rühren während 20 Stunden auf 100° erhitzt. Danach wurde das Reaktionsgemisch am Rotationsverdampfer eingeengt, der Rückstand in $CH_2Cl_2$ aufgelöst und mit NaOH-Lösung behandelt. Die organische Phase wurde mit Wasser gewaschen und über $Na_2So_4$ getrocknet. Der nach Einengen der organischen Phase erhaltene Rückstand wurde aus Methanol umkristallisiert. Man erhielt 21,1 g (RS)-(6,6'-Dimethylbiphenyl-2,2'-diyl)bis(phosphonsäure-diphenylester) als hellbeiges Pulver.

b) In einem 1 l Sulfierkolben, versehen mit Kühler, Thermometer, Rührer und Aufsatz für Inertgasbehandlung, wurden unter Argon 2,48 g (0,102 Mol) Mg-Späne mit 10 ml Tetrahydrofuran überschichtet und langsam unter Rühren mit einer Lösung von 15,13 g (92,8 mMol) 2-Bromthiophen in 20 ml Tetrahydrofuran versetzt, wobei die Reaktionstemperatur zwischen 40 und 50° gehalten wurde. Die Grignard-Lösung wurde mit 50 ml Tetrahydrofuran verdünnt, in einen Tropftrichter überführt und zu einer Lösung von 10 g (15,46 mMol) (RS)-(6,6'-Dimethylbiphenyl-2,2'-diyl) bis(phosphonsäurediphenylester) in 60 ml Tetrahydrofuran getropft. Nach 16 Stunden bei 60° wurde die erhaltene Suspension filtriert und das Filtrat mit wässriger $NH_4Cl$-Lösung hydrolysiert. Die wässrige Phase wurde abgetrennt und die organische Phase nacheinander mit ges. Natronlauge und ges. NaCl-Lösung gewaschen. Nach Trocknen über $Na_2SO_4$ wurde eingedampft und das erhaltene Rohprodukt aus $CH_2Cl_2$/EtOH (1/1) umkristallisiert. Man erhielt 5,63 g (RS)-(6,6'-Dimethylbiphenyl-2,2'-diyl)bis(di-2-thienylphosphinoxid) als fahlweisses Kristallpulver.

<u>Beispiel 5</u>

In zu Beispiel 4 analoger Weise wurden folgende Verbindungen hergestellt:

(RS)-(6,6'-Dimethylbiphenyl-2,2'-diyl)bis(di-2-furylphosphinoxid). $^1$H-NMR (250 MHz, $CDCl_3$): 7,60-7,26 (m,6 aromat. und 4 heteroaromat. H), 6,95, 6,90, 6,41, 6,30 (4m, je 2 heteroaromat. H), 1,87 (s, 2 aromat. $CH_3$).

(RS)-(6,6'-Dimethylbiphenyl-2,2'-diyl)bis(di-2-furylphosphin). IR (KBr): 1546 (Ar), 788 (1,2,3-trisubst. Benzol).

$^1$H-NMR (250 MHz, $CDCl_3$): 7,71-7,10 (m, 6 aromat. und 4 heteroaromat. H); 6,67, 6,41, 6,33, 6,27 (4m, je 2 heteroaromat. H); 1,51 (s, 2 aromat. $CH_3$). MS: 345 (100, $M^+$-$P(C_4H_3O)_2$).

<u>Beispiel 6</u>

In zu Beispiel 4b) analoger Weise wurden folgende Verbindungen hergestellt:

(R)-(6,6'-Dimethylbiphenyl-2,2'-diyl)bis(di-2-thienylphosphin). Smp. 152-154°; $[\alpha]_D^{20}$= +163 (c = 1, $CHCl_3$).
(S)-(6,6'-Dimethylbiphenyl-2,2'-diyl)bis(di-2-thienylphosphin). Smp. 153-154°; $[\alpha]_D^{20}$ = -161,6 (c = 1, $CHCl_3$).

Das hierbei jeweils als Ausgangsmaterial verwendete (R)- bzw. (S)-(6,6'-Dimethylbiphenyl-2,2'-diyl)bis(di-2-thienylphosphinoxid) wurde wie folgt hergestellt:

a) In einem 250 ml Rundkolben wurden 5,46 g (9,0 mMol) (RS)-(6,6'-Dimethylbiphenyl-2,2'-diyl)bis(di-2-thienylphosphinoxid) [hergestellt gemäss Beispiel 4] in 75 ml $CH_2Cl_2$ bei 40° gelöst und mit einer Lösung von 3,22 g (9,0 mMol) (-)-O,O'-Dibenzoyl-L-weinsäure in 55 ml Essigsäureäthylester versetzt. Nach 2-stündigem Rühren bei 0° wurde der ausgefallene Festkörper abfiltriert, zweimal mit je 10 ml Essigsäureäthylester gewaschen, in ca. 30 ml $CH_2Cl_2$ gelöst und mit 50 ml einer 1N NaOH-Lösung versetzt. Die organische Phase wurde zweimal mit Wasser gewaschen und anschliessend über $Na_2SO_4$ getrocknet. Nach Einengen der Lösung und Trocknen des Rückstandes bei ~10 Pa wurden 1,13 g (S)-(6,6'-Dimethylbiphenyl-2,2'-diyl)bis(di-2-thienylphosphin-**oxid**) erhalten als weisses Kristallpulver. $[\alpha]_D^{20}$ = +42,66 (c = 1, $CHCl_3$).

b) Das aus a) erhaltene Filtrat wurde analog wie dort beschrieben mit NaOH behandelt. Es resultierten 3,9 g (6,43 mMol) eines weissen Pulvers, welches in 25 ml $CH_2Cl_2$ bei 40° gelöst wurde und mit einer Lösung von 2,3 g (6,43 mMol) (+)-O,O'-Dibenzyol-D-weinsäure in 15 ml Essigsäureäthylester umgesetzt wurde. Die erhaltene klare Lösung wurde auf 0° abgekühlt und wie unter a) weiterbehandelt. Man erhielt 1,89 g (R)-(6,6'-Dimethylbiphenyl-2,2'-diyl) bis(di-2-thienylphosphin-**oxid**). $[\alpha]_D^{20}$ = -41,75 (c = 1, $CHCl_3$).

<u>Beispiel 7</u>

In zu den Beispielen 4 und 5 analoger Weise wurden folgende Verbindungen hergestellt:

(S)-(6,6'-Dimethylbiphenyl-2,2'-diyl)bis(di-2-furylphosphinoxid). $[\alpha]_D^{20}$ = +64,7 (c = 1, $CHCl_3$).

(S)-(6,6'-Dimethylbiphenyl-2,2'-diyl)bis(di-2-furylphosphin). Smp. 145-146°; $[\alpha]_{D,}^{20}$ = +88,6 (c = 1, $CHCl_3$).
(R)-(6,6'-Dimethylbiphenyl-2,2'-diyl)bis(di-2-furylphosphinoxid). $[\alpha]_{,D,}^{20}$ = -64,9 (c = 1, $CHCl_3$).
(R)-(6,6'-Dimethylbiphenyl-2,2'-diyl)bis(di-2-furylphosphin). Smp. 146-147°; $[\alpha]_{D,}^{20}$ = -88,8 (c = 1, $CHCl_3$).

Beispiel 8

a) Zu 14 ml 1,6N Butyllithium-Lösung in Hexan (19,6 mMol) wurde bei -78° eine Lösung von 3,35 g (25,0 mMol) Benzothiophen in 14 ml TFH via eine Kanüle innerhalb von wenigen Minuten einfliessen gelassen, wobei die Temperatur bis auf -45° anstieg. Man liess die entstehende weisse Suspension auf 0° erwärmen, kühlte auf -15°, rührte noch 20 Minuten bei dieser Temperatur und kühlte dann wiederum auf -78°. In die Suspension wurden 6,0 g (23,2 mMol) Magnesiumdibromid-Diätherat ($MgBr_2.Et_2O$) eingetragen, und man liess die Mischung auf RT erwärmen. Die entstandene Lösung wurde nach Verdünnen mit 20 ml THF via eine Kanüle bei -78° auf 0,90 g (2,0 mMol) (R) -(6,6'-Dimethoxybiphenyl-2,2'-diyl)bis(phosphonsäure-dichlorid) fliessen gelassen. Man liess anschliessend auf RT erwärmen und verdünnte nochmals mit 20 ml THF. Die gelb-braune Lösung wurde mit gesättigter $NH_4Cl$-Lösung und Essigsäureäthylester versetzt, die Phasen getrennt, und die organische Phase wurde mit Wasser und gesättigter Nacl-Lösung gewaschen, über $MgSO_4$ getrocknet, filtriert und eingedampft. Der Rückstand wurde in der minimalen Menge $CH_2Cl_2$ gelöst und an 50 g Kieselgel chromatographiert (Hexan/Essigsäureäthylester 1:1 und $CH_2Cl_2$/Essigsäureäthylester 2:8), wobei (R)-(6,6'-Dimethoxybiphenyl-2,2'-diyl)bis(dibenzo[b] thiophen-2-yl)phosphinoxid als beiges Pulver erhalten wurden. Umkristallisation aus $CH_2Cl_2$/Essigsäureäthylester lieferte 1,20 g (72%) beiges Pulver; Smp. 313-315°; $[\alpha]^{20}_{D}$ = +132,9 (c=1,0, $CHCl_3$).

In zum Vorhergehenden analoger Weise können auch folgende Verbindungen hergestellt werden:

(RS)-(6,6'-Dimethoxybiphenyl-2,2'-diyl)bis(dibenzo[b] thiophen-2-yl)-phosphinoxid, Smp. 293-295° (Thermoanalyse 292,9°),
(S)-(6,6'-Dimethoxybiphenyl-2,2'-diyl)bis(dibenzo[b] thiophen-2-yl)-phosphinoxid, Smp. 312-316°; $[\alpha]^{20}_{D}$ = -132,9 (c=1, $CHCl_3$).

b) Die gemäss a) erhaltenen Phosphinoxide wurden gemäss Beispiel 1b) reduziert und man erhielt folgende Verbindungen:

(R)-(6,6'-Dimethoxybiphenyl-2,2'-diyl)bis(dibenzo[b]thiophen-2-yl)-phosphin, Smp. 252-254°; $[\alpha]^{20}_{D}$ = +94,9 (c=1, $CHCl_3$),
(S)-(6,6'-Dimethoxybiphenyl-2,2'-diyi)bis(dibenzo [b] thiophen-2-yl)-phosphin, Smp. 252-253°; $[\alpha]^{20}_{D}$ = -93,4 (c=1, $CHCl_3$),
(RS)-(6,6'-Dimethoxybiphenyl-2,2'-diyl)bis(dibenzo[b]thiophen-2-yl)-phosphin.

Das gemäss a) als Ausgangsmaterial verwendete (R)-(6,6'-Dimethoxybiphenyl-2,2'-diyl)bis(phosphonsäure-dichlorid wurde wie folgt hergestellt:

Eine Lösung von 4,86 g (10,0 mMol) (R)-(6,6'-Dimethoxybiphenyl-2,2'-diyl)bis(phosphonsäure-diäthylester [Smp. 125-126°; $[\alpha]^{20}_{D}$ = +32,7 (c=1, $CHCl_3$) und hergestellt in Analogie zur Herstellung des entsprechenden Diphenylesters gemäss Beispiel 1)], 10 ml (16,33 g, 137 mMol) Thionylchlorid und 1,0 ml trockenem N,N-Dimethylformamid wurde während 6 Stunden unter Argon am Rückfluss gekocht. Das überschüssige Thionylchlorid wurde anschliessend abdestilliert und der Rückstand am Hochvakum (~10 Pa) bei 100° während 1 Stunde getrocknet. Das erhaltene viskose Oel wurde in 30 ml $CH_2Cl_2$ aufgenommen. Nach Filtration von wenig ungelöstem Material wurde das Filtrat mit 40 ml Aether versetzt, wobei ein weisses Pulver ausfiel. Filtration, Waschen mit Aether und Trocknen am Hochvakuum (~10 Pa) lieferte 3,10 g (R)-(6,6'-Dimethoxybiphenyl-2,2'-diyl)bis(phosphonsäure-dichlorid) vom Smp. 172-178°; $[\alpha]^{20}_{D}$ = +51,3 (c=1, $CHCl_3$).

In zum Vorhergehenden analoger Weise wurden folgende Verbindungen hergestellt:

(S)-(6,6'-Dimethoxybiphenyl-2,2'-diyl)bis(phosphonsäure-dichlorid), Smp. 172-178°; $[\alpha]^{20}_{D}$ = -50,2 (c=1, $CHCl_3$),
(RS)-(6,6'-Dimethoxybiphenyl-2,2'-diyl )bis(phosphonsäure-dichlorid), Smp. 195-198°.

Beispiel 9

In zu Beispiel 8 analoger Weise können auch folgende Verbindungen hergestellt werden:

(R)-(6,6'-Dimethoxybiphenyl-2,2'-diyl)bis(dibenzo[b]furan-2-yl)phosphinoxid, Smp. 288-292°; $[\alpha]_D^{20}$ = +82,3 (c=1, $CHCl_3$),
(R)-(6,6'-Dimethoxybiphenyl-2,2'-diyl)bis(dibenzo[b]furan-2-yl )phosphin, Smp. 239-241°; $[\alpha]_D^{20}$ = +31,6 (c=1, $CHCl_3$),
(S)-(6,6'-Dimethoxybiphenyl-2,2'-diyl)bis(dibenzo[b]furan-2-yl)phosphinoxid, Smp. 287-289°; $[\alpha]_D^{20}$ = -80,8 (c=1, $CHCl_3$),
(S)-(6,6'-Dimethoxybiphenyl-2,2'diyl)bis(dibenzo[b]furan-2-yl)phosphin, Smp. 239-240°; $[\alpha]_D^{20}$ = -30,6 (c=1, $CHCl_3$),
(RS)-(6,6'-Dimethoxybiphenyl-2,2'-diyl)bis(dibenzo[b]furan-2-yl)phosphinoxid,
(RS)-(6,6'-Dimethoxybiphenyl-2,2'-diyl)bis(dibenzo[b]furan-2-yl )phosphin,
(RS)-(6,6'-Dimethoxybiphenyl-2,2'-diyl)bis [bis(5-methylfuran-2-yl)phosphinoxid, Smp. 201-204°,
(RS)-(6,6'-Dimethoxybiphenyl-2,2'-diyl)bis [bis(5-methylfuran-2-yl)-phosphin, Smp. 159-160°,
(R)-(6,6'-Dimethoxybiphenyl-2,2'-diyl)bis [bis(5-methylfuran-2-yl)-phosphinoxid, Smp. 231-234°; $[\alpha]_D^{20}$ = -87,8 (c=1, $CHCl_3$),
(R)-(6,6'-Dimethoxybiphenyl-2,2'-diyl)bis [bis(5-methylfuran-2-yl). phosphin, Smp. 146-150°; $[\alpha]_D^{20}$ = +20,6 (c=1, $CHCl_3$),
(S)-(6,6'-Dimethoxybiphenyl-2,2'-diyl)bis [bis(5-methylfuran-2-yl)-phosphinoxid, Smp. 229-233°; $[\alpha]_D^{20}$ = -72,4 (c=1, $CHCl_3$),
(S)-(6,6'-Dimethoxybiphenyl-2,2'-diyl)bis [bis(5-methylfuran-2-yl)-phosphin, Smp. 146-150°; $[\alpha]_D^{20}$ = -20,1 (c=1, $CHCl_3$),
(RS)-(6,6'-Dimethoxybiphenyl-2,2'-diyl) (bis[bis(1-methylpyrrol-2-yl)-phosphinoxid], Smp. 297-299°,
(RS)-(6,6'-Dimethoxybiphenyl-2,2'-diyl)(bis[bis(1-methylpyrrol-2-yl)-phosphin], Smp. 162-166°,
(R)-(6,6'-Dimethoxybiphenyl-2,2'-diyl)(bis [bis(1-methylpyrrol-2-yl)-phosphinoxid], Smp. 298-302°; $[\alpha] = {}_D^{20}$ +12,4 (c=1, $CHCl_3$),
(R)-(6,6'-Dimethoxybiphenyl-2,2'-diyl)(bis[bis(1-methylpyrrol-2-yl)-phosphin],
(S)-(6,6'-Dimethoxybiphenyl-2,2'-diyl) (bis [bis(1-methylpyrrol-2-yl)-phosphinoxid],
(S)-(6,6'-Dimethoxybiphenyl-2,2'-diyl) (bis[bis(1-methylpyrrol-2-yl)-phosphin].

Beispiel 10

a) In einer Glove-Box ($O_2$-Gehalt <1 ppm) wurden in einem 50 ml Messkolben 9,7 mg (0,02 mMol) Dichloro-bis-(1,5-cyclooctadien)dirhodium und 22,7 mg (0,04 mMol) (S)-(6,6'-Dimethylbiphenyl-2,2'-diyl)bis(di-2-thienylphosphin) in 50 ml Toluol unter Rühren bei Raumtemperatur gelöst. Innert 10 Minuten bildete sich eine orangerote, klare Katalysatorlösung.

b) In einer Glove-Box ($O_2$-Gehalt <1 ppm) wurde ein 500 ml-Autoklav mit 16,11 g (78,85 mMol) (E)-3-(4-tert.Butylphenyl)-2-methylprop-2-en-1-ol, 145 ml Toluol und 5 ml Katalysatorlösung (gemäss a)) beladen. Dieses Gemisch wurde bei 100°, einem konstanten Druck von 60 bar $H_2$ und unter intensivem Rühren hydriert. Nach 22 Stunden betrug der Umsatz >99%. Die hellgelbe Hydrierlösung wurde aus dem Autoklaven gespült und am Rotationsverdampfer bei 60°/17 mbar eingedampft. Der Rückstand wurde bei 140°/0,01 mbar destilliert. Man erhielt 16,0 g (99,0%) (R)--3-(4-tert.Butylphenyl)-2-methylpropan-1-ol, als farbloses Oel, mit einer enantiomeren Reinheit von 91,3% e.e.

In zum Vorhergehenden analoger Weise wurde die Hydrierung durchgeführt mit einer aus (R)-(6,6'-Dimethylbiphenyl-2,2'-diyl)bis(di-2-thienylphosphin) hergestellten Katalysatorlösung. Die enantiomere Reinheit betrug in diesem Fall 90,2% e.e.

Beispiel 11

a) In einer Glove-Box ($O_2$-Gehalt <1 ppm) wurden in einem 100 ml Messkolben 11,2 mg (0,013 mMol) Tetra-μ-trifluoracetato-bis(1,5-cyclooctadien)-diruthenium(II) und 13,2 mg (0,026 mMol) (S)-(6,6'-Dimethylbiphenyl-2,2'-diyl)bis(di-2-furylphosphin) in 100 ml Methanol unter Rühren bei Raumtemperatur gelöst. Innert 16 Stunden

bildete sich eine orangerote, klare Katalysatorlösung.

b) In einer Glove-Box ($O_2$-Gehalt <1 ppm) wurde ein 500 ml-Autoklav mit 24,0 g (155,6 mMol) ((E)-3,7-Dimethylocta-2,6-dien-1-ol), 22 ml Methanol und 100 ml Katalysatorlösung beladen. Dieses Gemisch wurde bei 20°, einem konstanten Druck von 60 bar $H_2$ und unter intensivem Rühren hydriert. Nach 48 Stunden betrug der Umsatz 100%. Die hellgelbe Hydrierlösung wurde aus dem Autoklaven gespült und am Rotationsverdampfer bei 60°/17 mbar eingedampft. Der Rückstand wurde bei 65°/0,01 mbar destilliert. Man erhielt 23,8 g ((R)-3,7-Dimethyloct-6-en-1-ol, als farbloses Oel, mit einer Enantiomeren-Reinheit von 98,4% e.e.

Beispiel 12

In zu Beispiel 9 analoger Weise wurde ((E)-3,7-Dimethylocta-2,6-dien-1-ol) in Gegenwart einer aus (S)-(6,6'-Dimethylbiphenyl)bis(di-2-thienylphosphin) hergestellten Katalysatorlösung hydriert. Die Enantiomeren-Reinheit betrug in diesem Fall 98,8% e.e.

## Patentansprüche

1. Racemische und optisch aktive Phosphorverbindungen der allgemeinen Formel

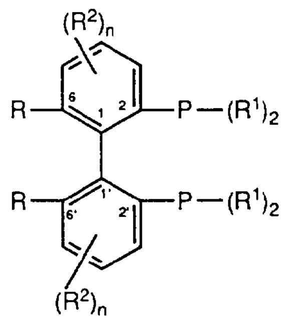

I

worin R niederes Alkyl, niederes Alkoxy **jeweils mit 1 bis 4 Kohlenstoffatomen** oder eine geschützte Hydroxygruppe bedeutet, $R^1$ einen fünfgliedrigen Heteroaromaten **der allgemeinen Formel**

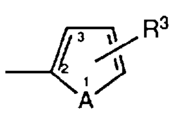 (a) 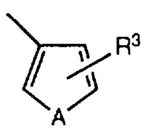 (b) 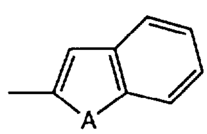 (c) 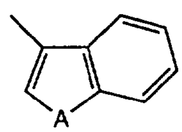 (d)

bedeutet, $R^2$ für niederes Alkyl oder niederes Alkoxy **jeweils mit 1 bis 4 Kohlenstoffatomen** steht; und n die Zahl 0, 1 oder 2 darstellt; **und in den Substituenten der Formeln (a) bis (d) A Sauerstoff Schwefel oder -$NR^4$; $R^3$ Wasserstoff niederes Alkyl oder niederes Alkoxy jeweils mit 1 bis 4 Kohlenstoffatomen; und $R^4$ niederes Alkyl mit 1 bis 4 Kohlenstoffatomen bedeuten.**

2. Phosphorverbindungen gemäss Anspruch 1 in der (R)- oder (S)-Form.

3. Phosphorverbindungen gemäss Anspruch 1 oder 2, worin n für die Zahl 0 steht.

4. Phosphorverbindungen gemäss einem der Ansprüche 1 bis 3, worin R Methoxy oder Methyl bedeutet.

5. Phosphorverbindungen gemäss einem der Ansprüche 1 bis 4, worin $R^1$ 2-Furyl, 3-Furyl, 2-Thienyl, 5-Methylfuran-2-yl, 2-Benzo[b]furanyl, Dibenzo[b]-thiophen-2-yl oder 1-Methylpyrrol-2-yl darstellt.

6. (RS)-, (R)- oder (S)-Dimethoxybiphenyl-2,2'-diyl)bis(di-2-furylphosphin).

7. (RS)-, (R)- oder (S)-Dimethoxybiphenyl-2,2'-diyl)bis(di-2-thienylphosphin).

8. (RS)-, (R)- oder (S)-Dimethylbiphenyl-2,2'-diyl)bis(di-2-furylphosphin).

**9.** (RS)-, (R)- oder (S)-Dimethylbiphenyl-2,2'-diyl)bis(di-2-thienylphosphin).

**10.** Verfahren zur Herstellung von racemischen bzw. optisch aktiven Phosphorverbindungen der allgemeinen Formel

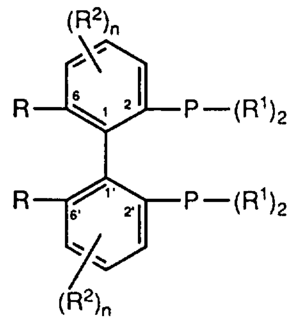

I

worin R niederes Alkyl, niederes Alkoxy **jeweils mit 1 bis 4 Kohlenstoffatomen** oder eine geschützte Hydroxygruppe bedeutet, $R^1$ einen fünfgliedrigen Heteroaromaten **der allgemeinen Formel**

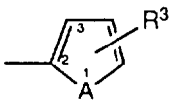 (a) 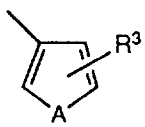 (b) 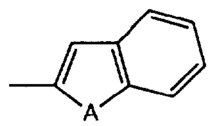 (c) 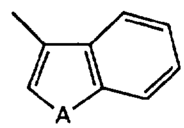 (d)

bedeutet, $R^2$ für niederes Alkyl oder niederes Alkoxy **jeweils mit 1 bis 4 Kohlenstoffatomen** steht; und n die Zahl 0, 1 oder 2 darstellt; **und in den Substituenten der Formeln (a) bis (d) A Sauerstoff Schwefel oder -NR⁴; R³ Wasserstoff niederes Alkyl oder niederes Aloxy jeweils mit 1 bis 4 Kohlenstoffatomen; und R⁴ niederes Alkyl mit 1 bis 4 Kohlenstoffatomen bedeuten,**

dadurch gekennzeichnet, dass man eine racemische oder optisch aktive Verbindung der allgemeinen Formel

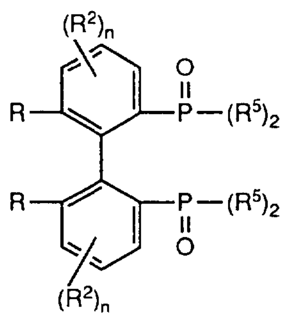

II

worin R, $R^2$ und n die obige Bedeutung haben und $R^5$ niederes Alkoxy mit 1 bis 4 Kohlenstoffatomen Phenoxy, Benzyloxy, Chlor oder Brom bedeutet, mit einer Verbindung der Formel

$$R^1MgX \text{ oder } R^1Li$$

worin $R^1$ die obige Bedeutung hat und X Chlor, Brom oder Jod darstellt,

zu einer Verbindung der Formel

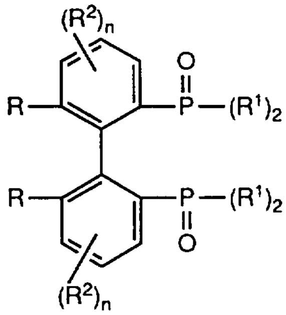

III

worin R, $R^1$, $R^2$ und n die obige Bedeutung haben,

umsetzt, und diese, gegebenenfalls nach Spaltung mittels O,O'-Dibenzoylweinsäure oder O,O'-Di-p-Toluylweinsäure in die (R)- und (S)-Form, zu einer Verbindung der Formel I reduziert.

## Claims

1. Racemic and optically active phosphorus compounds of the general formula

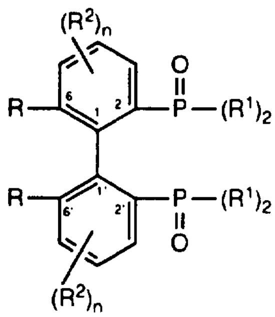

I

wherein R signifies lower alkyl, lower alkoxy each with 1 to 4 carbon atoms or a protected hydroxy group, $R^1$ signifies a five-membered heteroaromatic of the general formula

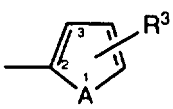
(a)

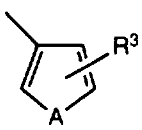
(b)

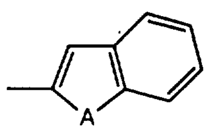
(c)

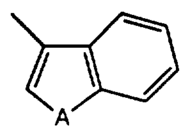
(d)

$R^2$ stands for lower alkyl or lower alkoxy each with 1 to 4 carbon atoms and n represents the number 0, 1 or 2; and in the substituents of formulae (a) to (d) A signifies oxygen, sulphur or $-NR^4$; $R^3$ signifies hydrogen, lower alkyl or lower alkoxy each with 1 to 4 carbon atoms; and $R^4$ signifies lower alkyl with 1 to 4 carbon atoms.

2. Phosphorus compounds in accordance with claim 1 in the (R) or (S) form.

3. Phosphorus compounds in accordance with claim 1 or 2, wherein n stands for the number 0.

4. Phosphorus compounds in accordance with any one of claims 1 to 3, wherein R signifies methoxy or methyl.

5. Phosphorus compounds in accordance with any one of claims 1 to 4, wherein $R^1$ signifies 2-furyl, 3-furyl, 2-thienyl, 5-methylfuran-2-yl, 2-benzo[b]furanyl, dibenzo[b]thiophen-2-yl or 1 -methylpyrrol-2-yl.

6. (RS)-, (R)- or (S)-Dimethoxybiphenyl-2,2'-diyl)bis(di-2-furylphosphine).

7. (RS)-, (R)- or (S)-Dimethoxybiphenyl-2,2'-diyl)bis(di-2-thienylphosphine).

8. (RS)-, (R)- or (S)-Dimethylbiphenyl-2,2'-diyl)bis(di-2-furylphosphine).

**9.** (RS)-, (R)- or (S)-Dimethylbiphenyl-2,2'-diyl)bis(di-2-thienylphosphine).

**10.** A process for the manufacture of racemic or optically active phosphorus compounds of the general formula

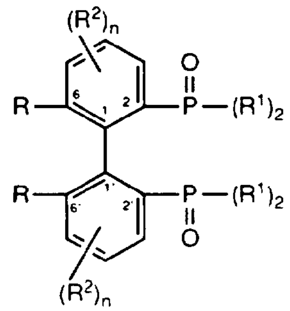

I

wherein R signifies lower alkyl, lower alkoxy each with 1 to 4 carbon atoms or a protected hydroxy group, $R^1$ signifies a five-membered heteroaromatic of the general formula

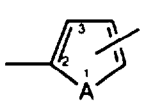

(a)

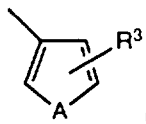

(b)

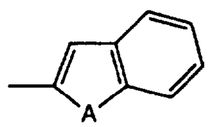

(c)

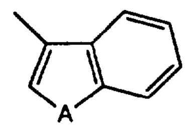

(d)

$R^2$ stands for lower alkyl or lower alkoxy each with 1 to 4 carbon atoms and n represents the number 0, 1 or 2; and in the substituents of formulae (a) to (d) A signifies oxygen, sulphur or $-NR^4$; $R^3$ signifies hydrogen, lower alkyl or lower

alkoxy each with 1 to 4 carbon atoms and $R^4$ signifies lower alkyl with 1 to 4 carbon atoms,

characterized by reacting a racemic or optically active compound of the general formula

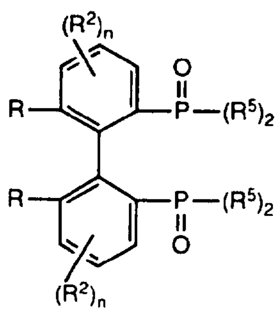

II

wherein R, $R^2$ and n have the above significance and $R^5$ signifies lower alkoxy with 1 to 4 carbon atoms, phenoxy, benzyloxy, chlorine or bromine,

with a compound of the formula

$$R^1MgX \text{ or } R^1Li$$

wherein $R^1$ has the above significance and X represents chlorine, bromine or iodine,

to give a compound of the formula

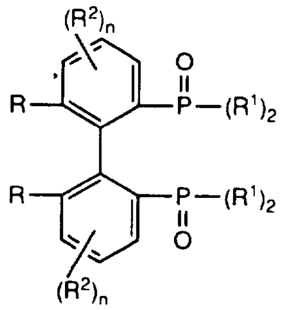

III

wherein R, $R^1$, $R^2$ and n have the above significance,

and reducing this, if desired after resolution into the (R) form and (S) form using O,O'-dibenzoyltartaric acid or O,O'-di-p-toluyltartaric acid, to a compound of formula 1.

## Revendications

1. Composés racémiques et optiquement actifs du phosphore, de formule générale

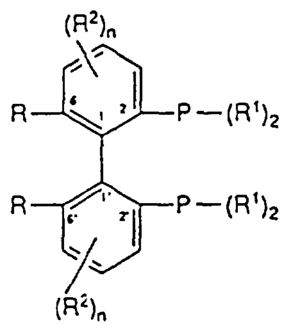

I

dans laquelle R est un radical alkyle inférieur, alcoxy inférieur, chacun ayant de 1 à 4 atomes de carbone, ou un groupe hydroxy protégé, $R^1$ est un radical hétéroaromatique à cinq chaînons de formule générale

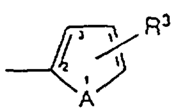 (a) 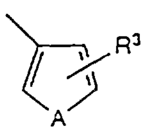 (b) 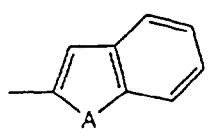 (c) 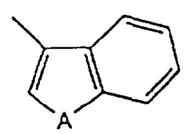 (d)

$R^2$ est un radical alkyle inférieur ou alcoxy inférieur, chacun ayant de 1 à 4 atomes de carbone ; et n est le nombre 0, 1 ou 2 ; et, dans les substituants des formules (a) à (d), A est l'oxygène, le soufre ou $-NR^4$; $R^3$ est un hydrogène, un radical alkyle inférieur ou alcoxy inférieur ayant chacun de 1 à 4 atomes de carbone ; et $R^4$ est un radical alkyle inférieur ayant de 1 à 4 atomes de carbone.

2. Composés du phosphore selon la revendication 1, sous la forme (R) ou (S).

3. Composés du phosphore selon la revendication 1 ou 2, dans lesquels n vaut 0.

4. Composés du phosphore selon l'une des revendications 1 à 3, dans lesquels R est le radical méthoxy ou méthyle.

5. Composés du phosphore selon l'une des revendications 1 à 4, dans lesquels $R^1$ est le radical 2-furyle, 3-furyle, 2-thiényle, 5-méthylfuranne-2-yle, 2-benzo-[b]furannyle, dibenzo[b]thiophène-2-yle ou 1-méthylpyrrol-2-yle.

6. (RS)-, (R)- ou -(S)-diméthoxybiphényle-2,2'-diyl)bis(di-2-furylphosphine).

7. (RS)-, (R)- ou -(S)-diméthoxybiphényle-2,2'-diyl)bis(di-2-thiénylphosphine).

8. (RS)-, (R)- ou -(S)-diméthylbiphényle-2,2'-diyl)-bis(di-2-furylphosphine).

**9.** (RS)-, (R)- ou -(S)-diméthylbiphényle-2,2'-diyl)-bis(di-2-thiénylphosphine).

**10.** Procédé pour préparer des composés racémiques et optiquement actifs du phosphore, de formule générale

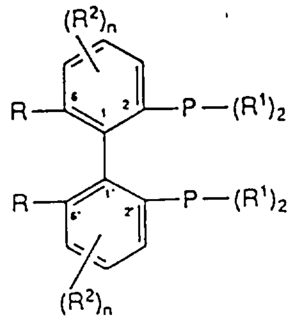

I

dans laquelle R est un radical alkyle inférieur, alcoxy inférieur, chacun ayant de 1 à 4 atomes de carbone, ou un groupe hydroxy protégé, $R^1$ est un radical hétéroaromatique à cinq chaînons de formule générale

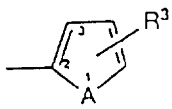 (a) 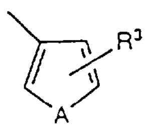 (b) 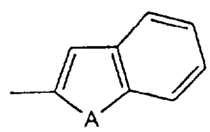 (c) 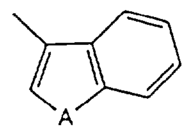 (d)

$R^2$ est un radical alkyle inférieur ou alcoxy inférieur, chacun ayant de 1 à 4 atomes de carbone ; et n est le nombre 0, 1 ou 2 ; et, dans les substituants des formules (a) à (d), A est l'oxygène, le soufre ou $-NR^4$; $R^3$ est un hydrogène, un radical alkyle inférieur ou alcoxy inférieur ayant chacun de 1 à 4 atomes de carbone ; et $R^4$ est un radical alkyle inférieur ayant de 1 à 4 atomes de carbone,

caractérisé en ce qu'on fait réagir un composé racémique ou optiquement actif de formule générale

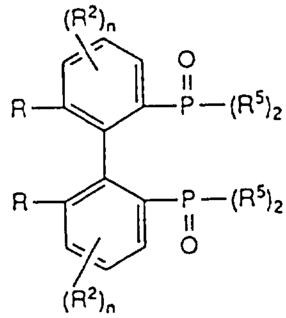

II

dans laquelle R, $R^2$ et n ont les significations ci-dessus, et $R^5$ est un radical alcoxy inférieur ayant de 1 à 4 atomes de carbone, phénoxy, benzyloxy, chloro ou bromo,
avec un composé de formule

$$R^1MgX \quad \text{ou} \quad R^1Li$$

où $R^1$ a les significations ci-dessus et X est le chlore, le brome ou l'iode,
pour obtenir un composé de formule

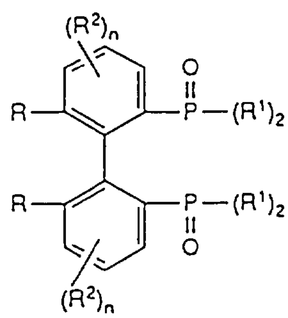

III

dans laquelle R, $R^1$, $R^2$ et n ont les significations données ci-dessus, et on réduit ce dernier, éventuellement après dissociation à l'aide d'acide O,O'-dibenzoyltartrique ou d'acide O,O'-di-p-toluyltartrique en la forme (R)

et (S), pour obtenir un composé de formule I.